# EUROPÄISCHE PATENTANMELDUNG

(11) **EP 1 752 116 A1**
(43) Veröffentlichungstag der Anmeldung: **14.02.2007**
(21) Anmeldenummer: 05405472.1
(22) Anmeldetag: 11.08.2005
(51) Int. Cl.: A61F 2/44

(54) **Intervertebralimplantat**

(71) Anmelder: Sepitec Foundation, 9490 Vaduz (LI)
(72) Erfinder: Magerl, Friedrich, 9011 St. Gallen (CH)
(74) Vertreter: Groner, Manfred

(57) **Zusammenfassung**

Das Intervertebralimplantat weist einen ersten Teil (2, 21, 26) und einen zweiten Teil (3, 22, 27) auf, die uniportal in einen Bandscheibenraum (B) implantierbar und in diesem miteinander verbindbar sind. Die Teile (2, 3; 21, 22; 26,27) weisen eine Öffnung (4, 5; 28, 29) zur Aufnahme von Spongiosa oder Knochenersatzmaterial auf. Die beiden genannten Teile (2, 3; 21, 22; 26, 27) besitzen Verbindungsmittel (8, 9; 23, 24; 31, 35), die beim Implantieren im Bandscheibenraum (B) ineinander greifen und hierbei die beiden Teile (2, 3; 21, 22; 26, 27) miteinander verbinden. Vorzugsweise weist wenigstens ein Teil (3, 22) eine Nut (8, 23) auf, in welche eine Feder (9, 24) des anderen Teils gleitverschieblich einfiihrbar ist. Die Nut (8, 23) und die Feder (9, 24) sind an inneren aneinander anzulegenden Schmalseiten (10, 11) der beiden Teile (2, 3; 21,22) angeordnet. Das Implantat ist uniportal durch eine transforaminale oder eine extraforaminale Öffnung (T, E) und damit vergleichsweise schonend implantierbar.

## Beschreibung

Die Erfindung betrifft ein Intervertebralimplantat für die Verbindung benachbarter Wirbelkörper, mit einem ersten Teil und einem zweiten Teil, die durch eine Öffnung (uniportal) in einen Bandscheibenraum einführbar und in diesem miteinander verbindbar sind und die jeweils einen zentralen Hohlraum zur Aufnahme von Spongiosa oder Knochenersatzmaterial aufweisen.

Intervertebralimplantate sind seit langem bekannt. Bekannt sind auch Intervertebralimplantate, die aus zwei Segmenten oder Teilen zusammengesetzt sind. Mit einem solchen vergleichsweise grossen Implantat ist es möglich, die Wirbel breiter abzustützen als dies mit einem kleineren Implantat möglich wäre. Bei einer solchen breiteren Abstützung kann der Wirbel weniger leicht über dem Implantat kippen. Zudem ist der Flächendruck, den das Implantat auf die Endplatten der Wirbelkörper ausübt, kleiner als bei einem schmaleren Implantat. Schliesslich ist ein solches Implantat weniger von der Konfiguration der Endplatten der Wirbelkörper abhängig.

Ein Intervertebralimplantat aus zwei in situ miteinander verbindbaren Teilen ist aus der WO 03/071992 bekannt geworden. Beide Teile sind als L-förmige Käfige ausgebildet und können jeweils in einer Ausnehmung Spongiosa oder ein Knochenersatzmaterial aufnehmen. Die beiden Teile müssen mit einem speziellen Instrument durch zwei Öffnungen (biportal) von hinten (posterior) in den Bandscheibenraum eingeführt werden. Es müssen somit links und rechts zwei Zugänge in den Bandscheibenraum und in die Bandscheibe gemacht werden, was eine grössere Destruktion der dorsalen Wirbelelemente sowie Traumatisierung des Spinalkanals bedeutet.

Durch die US 5,861,041 ist ein Intervertebralimplantat bekannt geworden, das ebenfalls aus zwei Teilen bzw. Segmenten besteht. In der Seitenansicht ist das Implantat keilförmig und in Draufsicht etwa elliptisch. Die beiden Teile werden nacheinander uniportal in den Bandscheibenraum eingeführt und liegen in Sagittalrichtung hintereinander. Damit sich die beiden Teile nicht voneinander lösen können, werden diese nach dem Implantieren miteinander verschraubt. Die beiden Teile können über einen hinteren Zugang uniportal eingesetzt werden und liegen schliesslich quer im Bandscheibenraum. Sie können aber auch über einen transforaminalen Zugang eingesetzt werden. Sie müssen dann jedoch in Querlage gedreht werden.

Der Erfindung liegt die Aufgabe zugrunde, ein Intervertebralimplantat der genannten Art zu schaffen, das noch einfacher und sicherer uniportal von hinten in den Bandscheibenraum einsetzbar ist.

Die Aufgabe ist bei einem erfindungsgemässen Intervertebralimplantat dadurch gelöst, dass die beiden genannten Teile Verbindungsmittel aufweisen, die beim Einführen des zweiten Teils in den Bandscheibenraum ineinander greifen und die beiden Teile miteinander verbinden. Beim erfindungsgemässen Intervertebralimplantat sind somit ein erster Teil und ein zweiter Teil vorgesehen, die uniportal transforaminal oder extraforaminal in den Bandscheibenraum einführbar sind. Beim Einführen des zweiten Teils in den Bandscheibenraum verbindet sich der zweite Teil in situ mit dem ersten Teil lösbar oder unlösbar. Durch das Zusammenfügen bzw. Verbinden der beiden Teile in situ entsteht insgesamt ein breites Implantat mit den oben genannten Vorteilen. Die Spongiosa bzw. das Knochenersatzmaterial kann bereits in den genannten Teilen vor dem Einsetzen eingefüllt werden. Die beiden Teile können keilförmig ausgebildet sein, so dass mit diesen der Bandscheibenraum erweitert werden kann, was eine stabile Verklemmung des Intervertebralimplantats und damit die Stabilität der Spondylodese fördert. Vor dem Einsetzen der beiden Teile wird der Bandscheibenraum mit an sich bekannten Distraktoren aufgedehnt. Die endgültige Distraktion erfolgt vorzugsweise durch die beiden Teile bzw. das Intervertebralimplantat selbst.

Nach einer Weiterbildung der Erfindung ist vorgesehen, dass wenigstens ein Teil eine Nut aufweist, in welcher eine Feder des anderen Teils gleitverschieblich einführbar ist. Dadurch können die beiden Teile beim Implantieren zusammengefügt und damit gegeneinander ausgerichtet sowie stabilisiert werden. Vorzugsweise sind die Nut und die Feder an inneren aneinander anzulegenden Schmalseiten der beiden Teile angeordnet. Beim Implantieren des zweiten Teils wird dieser am ersten bereits implantierte Teil geführt und kann damit besser als bisher in die gewünschte Position gebracht werden.

Nach einer Weiterbildung der Erfindung ist vorgesehen, dass die Nut und die Feder eine Schwalbenschwanzführung bilden. Dadurch wird erreicht, dass sich die beiden Teile in Sagittalrichtung nicht voneinander lösen können. Dadurch ergibt sich eine besonders stabile Verbindung zwischen den beiden Teilen. Die beiden Teile können trotzdem einfach und robust hergestellt werden.

Vorzugsweise sind die beiden Teile des Intervertebralimplantats ringförmig, insbesondere ovale Ringe und können zudem in der Querachse im Hinblick auf die Lordosierung in unterschiedlichem Ausmass keilförmig ausgebildet sein. Es können beide Teile offen oder auch ein Teil offen und einer geschlossen sein. Vorzugsweise sind die beiden Teile so in den Bandscheibenraum einführbar, dass sie in Sagittalrichtung hintereinander angeordnet sind. Beim Einführen der beiden Teile drehen sich diese von selbst von der Einführungsrichtung in die Endposition, wenn diese gemäss einer Weiterbildung der Erfindung Führungsmittel und insbesondere eine Führungskerbe, eine strukturierte Oberfläche und/oder scharfe Kanten aufweisen.

Nach einer Weiterbildung der Erfindung ist vorgesehen, dass die beiden Teile miteinander verriegelbar sind. Dadurch wird verhindert, dass die beiden Teile aneinander vorbeigleiten können. Die Verriegelung ist vorzugsweise so ausgebildet, dass die Verriegelung selbsttätig in situ erfolgt.

Weitere vorteilhafte Merkmale ergeben sich aus den abhängigen Patentansprüchen, der nachfolgenden Beschreibung sowie der Zeichnung.

Ausführungsbeispiele der Erfindung werden nachfolgend anhand der Zeichnung näher erläutert. Es zeigen:
- Figur 1: eine räumliche schematische Ansicht eines erfindungsgemässen Implantats,
- Figur 2: eine weitere räumliche Ansicht des Implantats gemäss Figur 1,
- Figur 3: eine Seitenansicht des Implantats,
- Figur 4: eine weitere Ansicht des Implantats,
- Figur 5: eine räumliche Ansicht des Implantats,
- Figur 6: eine räumliche Ansicht des anderen Teils des Implantats,
- Figur 7: eine Ansicht des einen Teils des Implantats,
- Fig.8-11: schematisch einzelne Phasen beim Verbinden der beiden Teile des Implantats und
- Fig. 12-15: schematisch einzelne Schritte beim Einführen und Verbinden der beiden Teile in den Bandscheibenraum.

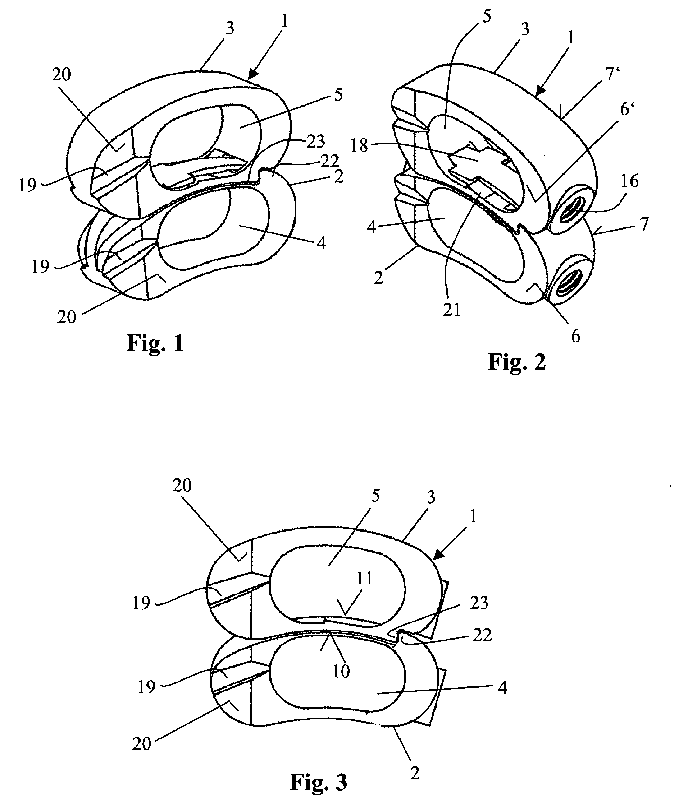

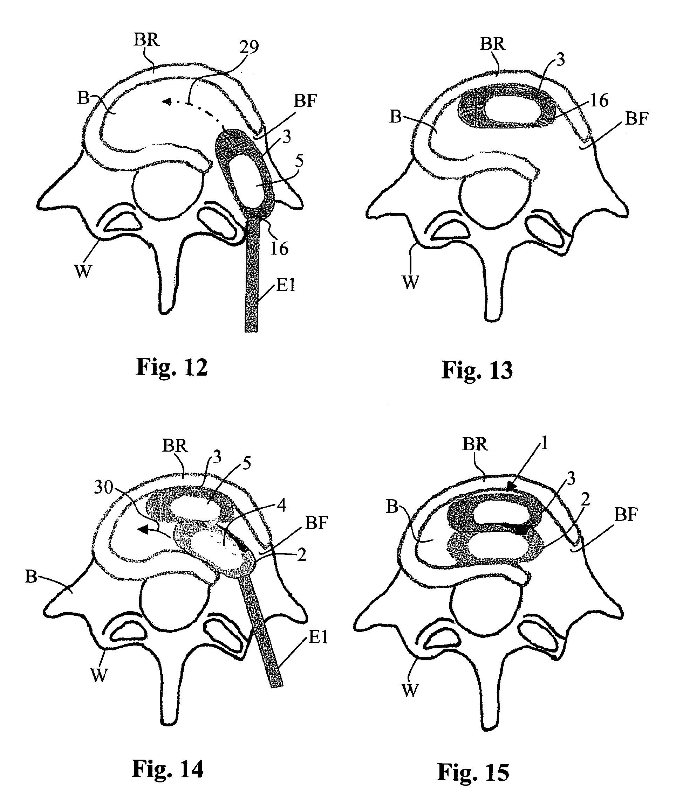

Das in den Figuren 1, 2 und 3 gezeigte Implantat 1 besteht aus zwei Teilen 2 und 3, die jeweils in der Ansicht gemäss Figur 3 bohnenförmig bzw. ringförmig ausgebildet sind und die zur Aufnahme von hier nicht gezeigter Spondiosa oder Knochenersatzmaterial einen auf zwei Seiten offenen durchgehenden Hohlraum 4 bzw. 5 aufweisen. Die beiden Teile 2 und 3 sind vorzugsweise jeweils einstückig aus einem geeigneten bio-kompatiblem Material, beispielsweise aus Titan oder einem verstärkten Kunststoff, beispielsweise kohlenfasernverstärktem PEEK hergestellt. Der Teil 2, der im implantierten Zustand gemäss Figur 15 der anteriore Teil ist, besitzt eine Schmalseite 11, die wenigstens bereichsweise an einer Schmalseite 10 des Teils 3 anliegt. Die Schmalseite 11 ist konvex und die Schmalseite 10 konkav ausgebildet. Beide Teile 2 und 3 besitzen zudem Oberflächen 6 und 7 bzw. 6' und 7'; die Oberflächen 6 und 6' als auch die Seitenflächen 7 und 7' liegen im Wesentlichen in der gleichen Fläche. Die Flächen 6, 6', 7 und 7' besitzen jeweils an einem Ende des Teils 2 bzw. des Teils 3 eine Distraktionsfläche, durch welche das entsprechende Ende keilförmig gebildet ist. Diese keilförmigen Abschrägungen ermöglichen ein Auseinanderdrücken der Wirbelkörper zur Vergrösserung der intervertebralen Distanz. Zudem sind im Bereich dieser Distraktionsflächen 20 Führungsrinnen 19 angeordnet, die Vertiefungen bilden und welche die beiden Teile 2 und 3 jeweils beim Einführen in den Bandscheibenraum führen. Es wird hierzu auf die Patentanmeldung PCT/EP 2004/002466 des Anmelders hingewiesen.

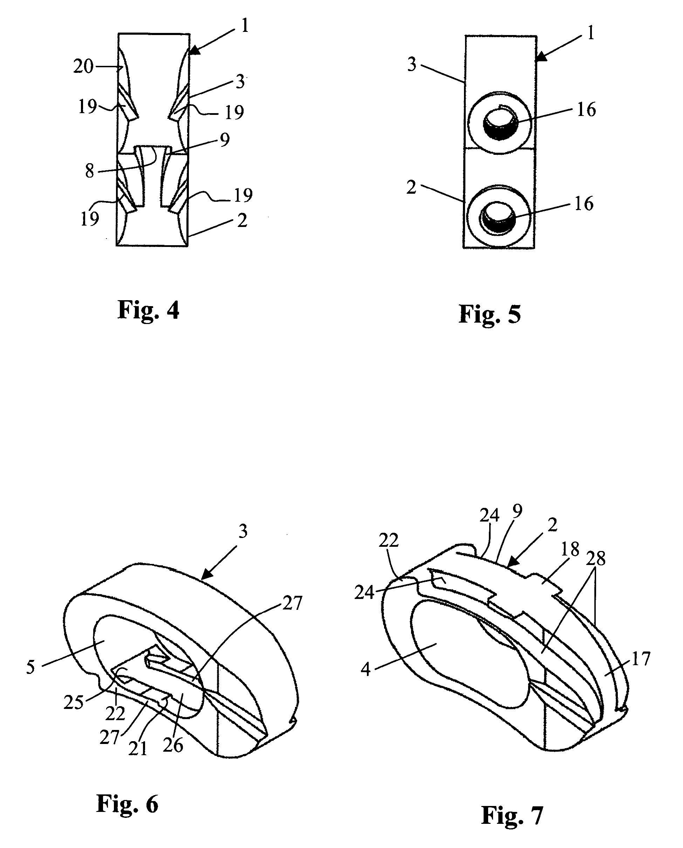

Der Teil 2 weist an der Schmalseite 11 eine Feder 9 auf, die sich im Wesentlichen über die gesamte Schmalseite 11 erstreckt. Die Feder 9 besitzt Oberflächen 24, die mit Ausnahme im Bereich eines Einführungsteiles 17 parallel zueinander verlaufen. Im Einführungsteil 17 laufen diese Oberflächen 24 keilförmig zusammen. In der in Figur 7 gezeigten Stellung ist am linken Ende der Feder 9 eine Schulter 22 angeordnet, die breiter ist als die Feder 9. Die Schulter 22 bildet einen Anschlag für den Teil 3, wie weiter unten näher erläutert wird. Zwischen der Schulter 22 und dem Einführteil 17 ist an der Feder 9 ein Verriegelungsteil 18 angeformt, der wie ersichtlich platten- oder flügelförmig ausgebildet ist und der beidseitig die Oberflächen 24 überragt.

Der Teil 3 besitzt an der Fläche 10 eine Einführöffnung 25, an die gemäss Figur 6 ein durchgehender Schlitz 26 angrenzt, die schliesslich gemäss Figur 4 in eine Nut 8 übergeht. Im Bereich der Öffnung 25 ist eine Schulter 22 angeformt, die mit der oben erwähnten Schulter 23 zusammenarbeitet. Seitlich neben dem Schlitz 26 verlaufen Stege 27, an denen innenseitig jeweils eine Stufe 21 angeformt ist, die mit dem Verriegelungsteil 18 zusammenarbeiten und mit diesem ein Verriegelungsorgan bilden. Der Schlitz 26 und die Öffnung 25 sind so ausgebildet, dass in diese die Feder 9 einführbar ist. Bei diesem Einführen bilden Gleitflächen 28, die gemäss Figur 7 neben der Feder 9 angeordnet sind, Anlageflächen an der Schmalseite 10 im Bereich der beiden Stege 27. Die Stege 27 sind im Wesentlichen etwa gleich breit wie die Gleitflächen 28. Die Krümmung der Gleitflächen 28 ist korrespondierend zur Krümmung der Schmalseite 10 ausgebildet, so dass sich ein flächiger Kontakt beim Aufschieben ergibt.

Die Oberflächen 24 sind nicht zwingend parallel zu einander, sondern können auch so geneigt sein, dass die Feder 9 im Querschnitt trapezförmig ist. Entsprechend ist der Schlitz 26 ausgebildet. In diesem Fall ergibt sich eine schwalbenschwanzförmige Ausführung.

Die Teile 2 und 3 besitzen jeweils eine Schraubenbohrung 16, an denen diese zum Implantieren mit einem hier nicht gezeigten Einführungsinstrument verbindbar sind. Solche Einführungsinstrumente sind dem Fachmann bekannt. Es wird hier wiederum auf die oben genannte PCT/EP 2004/002466 hingewiesen.

Die beiden Teile 2 und 3 können in situ im Bandscheibenraum B miteinander zu dem in den Figuren 1 bis 3 gezeigten Implantat 1 zusammengeführt und miteinander fest verbunden werden. Hierbei wird zuerst der Teil 2 bzw. der vordere Teil und anschliessend der Teil 3 bzw. der hintere Teil eingeführt, wie dies in den Figuren 12 bis 15 gezeigt ist. Der Teil 3 ist am implantierten Teil 2 geführt und verbindet sich zwangsläufig mit diesem. Nachfolgend wird die Herstellung dieser Verbindung anhand der Figuren 6 bis 11 näher erläutert.

Die Figuren 6 und 7 zeigen die beiden Teile 2 und 3 vor dem Implantieren. Das Implantieren der Teile 2 und 3 erfolgt durch eine Öffnung BF im Bandscheibenraum B und damit uniportal und vorzugsweise lateral wie in den Figuren 12 bis 15 gezeigt.

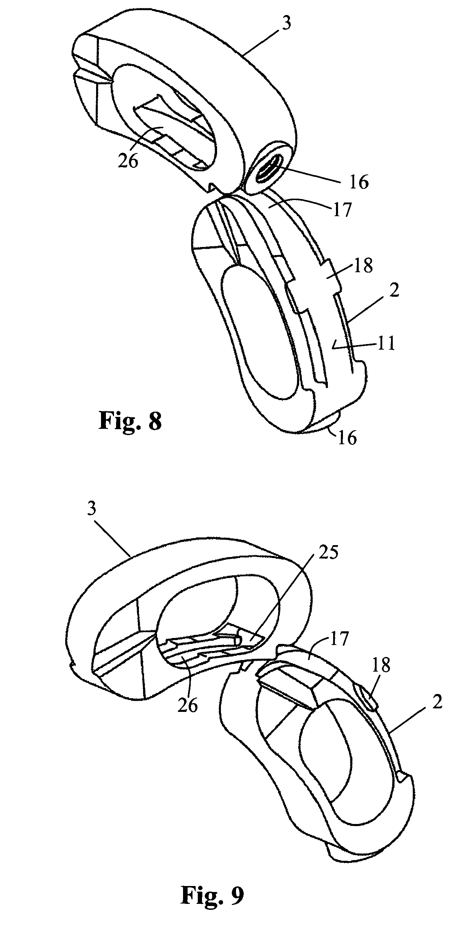

Anhand der Figuren 8 bis 11 wird nachfolgend das Verbinden der beiden Teile 2 und 3 zum Implantat 1 näher erläutert. Dieses Zusammenfügen erfolgt wie oben erwähnt in situ im Bandscheibenraum B.

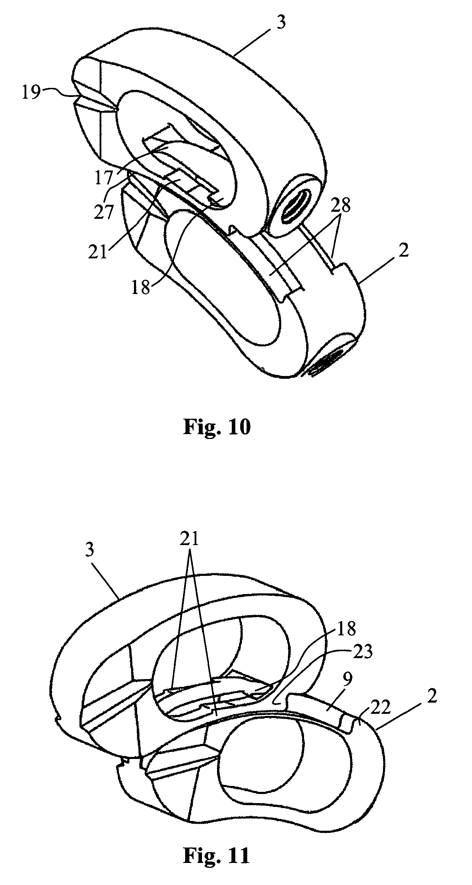

In der Anordnung gemäss Figur 3 wird davon ausgegangen, dass der Teil 3 mit einem hier nicht gezeigten Einführungsinstrumenten in den Bandscheibenraum B eingesetzt ist. Mit denselben Instrumenten wird der Teil 2 durch die gleiche Öffnung BF ebenfalls in den Bandscheibenraum B eingeführt. Wie die Figur 9 zeigt, wird der Teil 2 nun so geführt, dass der sich nach vorne keilförmig verjüngende Einführteil 17 durch die Öffnung 25 in den Schlitz 26 eingeführt werden kann. Die Figur 10 zeigt den Zustand, bei welchem sich dieser Einführteil 17 bereits in dem Schlitz 26 befindet. Wie ersichtlich, ist hierbei der Teil 2 mit den Gleitflächen 28 am Teil 3 geführt. Weitere Führungsmittel sind die Feder 9 mit dem Einführteil 17, welche den Stegen 27 entlang gleiten. Bei dem in Figur 10 gezeigten Zustand ist zudem der Verriegelungsteil 18 durch die Öffnung 25 in den Teil 3 eingeführt und liegt nun oberseitig auf den Stegen 27 auf. Der Teil 2 wird nun weiter in gleicher Richtung verschoben, wobei er wie erläutert am Teil gleitverschieblich geführt ist. Der Teil 2 gelangt nun über die in Figur 11 gezeigte Stellung in die in den Figuren 1 bis 3 gezeigte endgültige Stellung. Hierbei wird der Verriegelungsteil 18 über die rampenartig ansteigenden Stufen 21 geschoben und überspringt diese schliesslich. Dadurch wird der Teil 2 mit dem Teil 3 verrastet. Diese Verrastung kann lösbar oder auch unlösbar sein. Die Endposition wird durch einen Anschlag der Schultern 22 an den Schultern 23 bestimmt. Der Teil 2 ist damit mit dem Teil 3 verriegelt und diese Teile bilden damit das Implantat 1.

Die Verriegelung zwischen den beiden Teilen 2 und 3 kann auch mit anderen geeigneten Rastmitteln oder dergleichen erfolgen. Eine solche Verrastung bzw. Verriegelung ist jedoch nicht zwingend. Denkbar ist auch eine Ausführung, bei welcher die beiden Teile 2 und 3 nicht verriegelt werden. Bei einer solchen Ausführung sind die Nut 8 und der Schlitz 46 vorzugsweise als eine Art Schwalbenschwanznut ausgebildet, in welche die Federn 9 mit korrespondierendem Querschnitt eingesetzt ist. Dadurch ergibt sich eine Verbindung, welche die beiden Teile 2 und 3 in Sagittalrichtung fest miteinander verbindet.

Die Figuren 12 bis 15 zeigen schematisch das Einführen und Verbinden der beiden Teile 2 und 3 durch die Öffnung BF zur Bildung des Implantates 1 im Bandscheibenraum B.

Die Figur 12 zeigt den Teil 3, der mit einem Einführungsinstrument E1 in Richtung des Pfeiles 29 in den ausgeräumten Bandscheibenraum B eingeführt wird. Beim Einführen findet die oben erwähnte Distraktion statt. Zudem wird der Teil 3 wie ebenfalls oben erläutert im Bandscheibenraum B geführt. Die Führungselemente sind insbesondere die Führungsrinne 19 und scharfe Kanten und Oberflächenstrukturen gemäss der PCT/EP2004/002466. Schliesslich liegt der Teil 3 wie in Figur 13 gezeigt zwischen den beiden Wirbeln W, (von denen hier lediglich einer der Wirbel gezeigt ist) im Bandscheibenraum B in der vorgesehenen Position. Hierbei ist der Teil 3 bezüglich des Bandscheibenraumes B wie gezeigt ausgerichtet und liegt mit seiner konvexen Schmalseite an den Bandscheibenringen BR der beiden benachbarten Wirbeln W an.

Wie in Figur 14 gezeigt, wird nun der Teil 2 mit einem Einführungsinstrument E1 in den Bandscheibenraum B eingeführt. Der Teil 3 verbleibt hierbei in der in Figur 13 gezeigten Position. Der Teil 2 ist wie oben erläutert am Teil 3 geführt und wird weiter in Richtung des Pfeiles 30 vorgeschoben. Während dieser Bewegung wird die oben erwähnte Rastverbindung gebildet. Das damit gebildete und in Figur 15 gezeigte Implantat 1 bildet damit eine Einheit. Wie bereits erwähnt, können die beiden Teile 2 und 3 auch auf andere Weise verbunden sein, wobei eine Verrastung nicht zwingend ist. Denkbar ist auch eine Ausführung, bei welcher der Eingriff lediglich über eine Nut und eine Feder erfolgt. Die Nut kann eine Schwalbenschwanznut oder eine übliche Nut mit parallelen Seitenwänden sein.

### Bezugszeichenliste

- 1: Implantat
- 2: Teil
- 3: Teil
- 4: Hohlraum
- 5: Hohlraum
- 6: Oberfläche
- 7: Oberfläche
- 8: Nut
- 9: Feder
- 10: Schmalseite
- 11: Schmalseite

- 16: Schraubenbohrung
- 17: Einführteil
- 18: Verriegelungsteil
- 19: Führungsrinne
- 20: Distraktionsfläche

- 21: Stufe
- 22: Schulter
- 23: Schulter
- 24: Oberfläche
- 25: Öffnung
- 26: Schlitz
- 27: Stege
- 28: Gleitflächen
- 29: Pfeil
- 30: Pfeil

- B: Bandscheibenraum
- BF: Bandscheibenöffnung (Fenster)
- BR: Bandscheibenring
- E1: Einführungsinstrument

## Patentansprüche

1. Intervertebralimplantat für die Verbindung benachbarter Wirbelkörper (W), mit einem ersten Teil (3) und einem zweiten Teil (2), die durch eine Öffnung (BF) in einen Bandscheibenraum (B) einführbar und in diesem miteinander verbindbar sind und die jeweils einen zentralen Hohlraum (4, 5) zur Aufnahme von Spongiosa oder Knochenersatzmaterial aufweisen, **dadurch gekennzeichnet, dass** die beiden genannten Teile (2, 3) Verbindungsmittel (9; 26, 8, 18) aufweisen, die beim Einführen des zweiten Teils (2) in den Bandscheibenraum (B) ineinander greifen und hierbei die beiden Teile (2, 3) miteinander verbinden.

2. Implantat nach Anspruch 1, **dadurch gekennzeichnet, dass** wenigstens ein Teil (3) eine Nut (8) oder einen Schlitz (26) aufweist, in welche eine Feder (9) des anderen Teils gleitverschieblich einführbar ist.

3. Implantat nach Anspruch 2, **dadurch gekennzeichnet, dass** die Feder (9) an einer konvexen Schmalseite (11) des entsprechenden Teils (2, 3) angeordnet ist.

4. Implantat nach Anspruch 2 oder 3, **dadurch gekennzeichnet, dass** die beiden Teile (2, 3) mit einer Schwalbenschwanzführung verbindbar sind.

5. Implantat nach Anspruch 1, **dadurch gekennzeichnet, dass** der erste Teil (3) und der zweite Teil (2) Verbindungsmittel (18, 21) aufweisen, mit denen die beiden Teile (2, 3) miteinander verrastbar oder verriegelbar sind.

6. Implantat nach Anspruch 5, **dadurch gekennzeichnet, dass** als Verbindungsmittel ein Teil (2) ein Rast- oder Verriegelungsteil (18) aufweist, das durch eine Öffnung (25) des anderen Teils (3) in diesen einführbar ist.

7. Implantat nach einem der Ansprüche 1 bis 6, **dadurch gekennzeichnet, dass** die beiden Teile (2, 3) bohnenförmig ausgebildet sind.

8. Implantat nach einem der Ansprüche 1 bis 7, **dadurch gekennzeichnet, dass** der eine Teil (3) zwei im Abstand zueinander angeordnete Stege (27) aufweist, zwischen denen ein Schlitz (26) angeordnet ist, in welchen ein Teil (3) des anderen Teils (2) einführbar ist.

9. Implantat nach einem der Ansprüche 1 bis 8, **dadurch gekennzeichnet, dass** die beiden Teile (2, 3) in Sagittalrichtung hintereinander angeordnet sind.

10. Implantat nach einem Ansprüche 1 bis 9, **dadurch gekennzeichnet, dass** die beiden Teile (2, 3) miteinander in einer Endposition lösbar oder unlösbar miteinander verriegelbar oder verrastbar sind.

11. Implantat nach einem der Ansprüche 1 bis 10, **dadurch gekennzeichnet, dass** die beiden Teile (2, 3) jeweils wenigstens eine Schulter (22, 23) aufweisen, die beim Fügen der beiden Teile (2, 3) in der Endposition aneinander anliegen.
